# EUROPÄISCHE PATENTSCHRIFT

(11) **EP 4 124 326 B1**
(45) Veröffentlichungstag und Bekanntmachung des Hinweises auf die Patenterteilung: **09.09.2026**
(21) Anmeldenummer: 22187979.4
(22) Anmeldetag: 29.07.2022
(51) Int. Cl.: A61F 13/49, A61F 13/56, B32B 7/05, B32B 27/12

(54) **ELASTISCHES VLIESSTOFFLAMINAT MIT NICHT ELASTISCHEN, PARALLEL VERLAUFENDEN STREIFEN**
ELASTIC NONWOVEN LAMINATE WITH NON-ELASTIC, PARALLEL STRIPS
STRATIFIÉ NON TISSÉ ÉLASTIQUE POURVU DE BANDES PARALLÈLES NON ÉLASTIQUES

(30) Priorität: 29.07.2021 DE 102021119678
(43) Veröffentlichungstag der Anmeldung: 01.02.2023
(73) Patentinhaber: K.L. Kaschier- und Laminier GmbH, 48455 Bad Bentheim-Gildehaus (DE)
(72) Erfinder: Baldauf, Georg, 10405 Berlin (DE); Oberhaus, Christina, 48683 Ahaus (DE)
(74) Vertreter: Cohausz Hannig Borkowski Wißgott

(56) Entgegenhaltungen:
- EP-B1- 2 234 571
- EP-B2- 1 267 787
- WO-A1-2021/226034
- US-A1- 2019 231 606
- US-A1- 2020 268 563

## Beschreibung

Die Erfindung betrifft ein bahnförmiges Vliesstofflaminat zur Herstellung eines elastischen Verschlusselements einer Windel oder eines elastisch formgebenden Elements am Rand einer Windel, mit wenigstens einer Laminatschicht aus Vliesstoff und einer daran anliegenden Laminatschicht aus einer elastischen Folie, wobei die Laminatschichten in einem gedehnten Zustand der Folie an zahlreichen Schweißpunkten miteinander verschweißt sind. Die Erfindung betrifft ferner die Verwendung eines erfindungsgemäßen Vliesstofflaminats zur Herstellung eines Windelverschlusselements.

Es ist beispielsweise der US-Patentanmeldung US 2020/0268563 A1 und der internationalen Anmeldung WO 2021/226034 A1 bekannt, ein bahnförmiges Vliesstofflaminat bei Windeln zu verwenden, wobei eine elastische Folie zwischen zwei Vliesstoffen liegt und die so gebildeten drei Laminatschichten über Schweißpunkte miteinander verschweißt werden. Bei Verwendung eines solchen Vliesstofflaminats bei Windeln ist es wichtig, dass das Hygieneprodukt sich einfach herstellen lässt und die seitlichen Randbereiche des Laminats leicht und sicher aufeinander befestigbar sind.

Aufgabe der Erfindung ist es, eine hohe Prozesssicherheit bei der Verarbeitung und Befestigung des Laminats zu elastischen Windelverschlusselementen oder elastisch formgebenden Elementen am Rand einer Windel zu erreichen. Auch ist es Aufgabe der Erfindung, in dem Endprodukt einen Eindruck von Weichheit und Drapierfähigkeit zu vermitteln und dessen Reißfestigkeit zu verbessern.

Diese Aufgaben werden durch ein Vliesstofflaminat mit den Merkmalen des Anspruchs 1 gelöst.

Vorteilhafte Ausgestaltungen des erfindungsgemäßen Vliesstofflaminats sind in den Unteransprüchen aufgeführt.

Die Folie ist beim Verschweißen in einem mittleren Bereich quer zur Längsrichtung des Vliesstofflaminat gedehnt und an den beiden seitlichen Längsrändern in einer Breite von jeweils 5 - 25 % der Gesamtbreite der Folie nicht gedehnt, so dass das Vliesstofflaminat im entspannten Zustand der Folie im mittleren Bereich Falten im Vliesstoff wirft, die sich in Längsrichtung des Vliesstofflaminats erstrecken, und im Bereich der Längsränder keine Falten im Vliesstoff aufweist, sondern dort zwei parallel verlaufende, nicht elastische Versteifungszonen bildet, wobei die Folie im Bereich der Längsränder eine glatte, geschlossene Oberflächenstruktur und im mittleren Bereich eine faltige, gefurchte Oberflächenstruktur aufweist.

Der Vorteil der Ausführung eines elastischen Laminates mit zwei an den elastischen Bereich angrenzenden, parallel verlaufenden, nicht elastischen Längsrändern ist die höhere Prozesssicherheit bei der Verarbeitung des Laminats zu elastischen Windelverschlusselementen und deren einfachere und sichere Befestigung am Windelchassis, wie nachfolgend deutlich wird.

Die Verarbeitung des erfindungsgemäßen Vliesstofflaminats zu Windelverschlusselementen erfolgt durch die Schritte:
- Transportieren des Vliesstofflaminats in Längsrichtung zu einer ortsfesten ersten Station, an der wiederholt Klebverschlüsse an einem der nicht elastischen Längsränder des Vliesstofflaminats fahnenartig von diesem abstehend angebracht werden, so dass eine Serie von zueinander beabstandeten Klebverschlüssen vorliegt,
- Transportieren des Vliesstofflaminats in Längsrichtung zu einer ortsfesten zweiten Station, an der die gewünschten Verschlusselemente wiederholt aus dem Vliesstofflaminat so ausgestanzt werden, dass jedes Verschlusselement einen der Klebverschlüsse umfasst und
- Befestigung der Verschlusselemente mit ihrem dem Klebverschluss gegenüberliegenden, nicht elastischen Längsrand an einem Windelchassis, insbesondere mit Hilfe von Klebstoff und/oder einer Verpressung und/oder Ultraschallschweißen.

Bei beiden Befestigungsprozessen, d.h. für die Anbringung der Klebverschlüsse am Vliesstofflaminat einerseits und die Anbringung des aus diesem ausgestanzten Verschlusselements an einem Windelchassis, ist eine feste, nicht weich elastisch verformbare Laminatzusammensetzung in der Befestigungszone von Vorteil. Aus diesem Grund ist erfindungsgemäß vorgesehen, dass die nicht elastischen Längsränder des Vliesstofflaminats die Befestigungszonen bilden.

Ein weiterer Vorteil des erfindungsgemäßen Vliesstofflaminats besteht darin, dass durch die erhöhte Steifigkeit der Längsränder gegenüber dem elastischen mittleren Bereich eine verbesserte Verarbeitbarkeit auf einer Windelmaschine erreicht wird, auf der die o.g. Schritte ausgeführt werden, da das Laminat eine geringere Rollneigung aufweist, d.h. weniger dazu tendiert, sich zur Bahnmitte hin zusammenzurollen.

In einer Ausführungsvariante weist das Vliesstofflaminat nur eine Laminatschicht aus Vliesstoff auf. Vorzugsweise weist es jedoch zwei Laminatschichten aus Vliesstoff auf, wobei die Folie dazwischen liegt. Somit sind beide Seiten des Laminats aus Vliesstoff und damit weich und anschmiegsam, was gerade bei Hygieneprodukten gewünscht ist.

Die Folie ist eine Coextrusionsfolie mit einer Schichtstruktur der Art A/B/A, wobei die Dicke der Schicht A zwischen 2% und 14 % der Gesamtdicke der Folie beträgt. Dies verbessert die Reißfestigkeit.

Die Folie kann 10% bis 100 % der gesamten Fläche im Vliesstofflaminat einnehmen, bevorzugt 25% bis 50%.

Die Schweißpunkte sind bevorzugt in sinusförmigen Reihen angeordnet, deren Amplituden beispielhaft das 0,2 bis 0,5-fache des Abstands der Reihen voneinander in Längsrichtung des Vliesstofflaminats betragen kann. Durch die Bildung sinusförmiger Reihen können entlang einer Reihe mehr Schweißpunkte bei gleichem Abstand zu einander angeordnet werden, als bei einer geraden Reihe, so dass mehr Befestigungspunkte die Laminatschichten zusammenhalten. Ein weiterer Vorteil der Verwendung sinusförmiger Reihen besteht darin, dass eine zur Erzeugung der Schweißpunkte verwendete Ultraschalleinrichtung weniger verschleißt. Hierzu sei veranschaulicht, dass die Schweißpunkte durch Schweißnoppen auf einer Schweißwalze erzeugt werden können, an denen das zu verschweißende Laminat anliegt, wobei der Schweißwalze gegenüberliegend eine Sonotrode angeordnet ist, die Ultraschallschwingungen an die Schweißnoppen heranführt, um diese zu erhitzen, wobei das Laminat zwischen Sonotrode und Schweißwalze liegt. Durch eine sinusförmige Anordnung der Schweißnoppen entlang der Walzenachse wird erreicht, dass der Sonotrode stets wenigstens eine Schweißnoppe gegenüberliegt, und nicht wie bei der Drehung einer Schweißwalze mit geraden Reihen von Schweißnoppen abwechselnd keine und alle Schweißnoppen gegenüberliegt.

Durch die sinusförmige Anordnung der Schweißnoppen wird nach der Relaxierung der Folie eine deutlich sichtbare, wellenförmige Struktur in dem Vliesstofflaminat geschaffen, das den Anschein gibt, dass ein daraus hergestelltes Produkt eine hohe Weichheit und eine optimale Drapierfähigkeit aufweist. Hierbei zeichnet sich das Verfahren durch einfache Herstellungsschritte und durch eine einfache Weiterverarbeitung zum Endprodukt aus.

Der Abstand der Schweißpunktreihen zueinander in Längsrichtung des Vliesstofflaminats kann zwischen 2mm und 20mm vorzugsweise zwischen 3mm bis 5mm betragen.

Die Schweißpunkte entlang der Reihen können mit einem Abstand zueinander von 2mm bis 4mm, vorzugsweise von 2,2 bis 2,6 mm erzeugt sein.

Die Laminatschicht oder -schichten aus Vliesstoff kann oder können jeweils ein Gewicht von 10g/m² bis 30 g/m² aufweisen.

Die Folie kann ein Gewicht von 25 g/m² bis 110 g/m² aufweisen.

Zum Herstellen des bahnförmigen Vliesstofflaminats kann die Folie im mittleren Bereich quer zur Längsrichtung des Vliesstofflaminats um das 1,5 bis 3-fache, insbesondere um das 2,6 bis 2,8-fache, gedehnt werden und an den beiden seitlichen Längsrändern in einer Breite von jeweils 5 - 25 % der Gesamtbreite der Folie nicht gedehnt werden, wobei die Laminatschichten anschließend in diesem Zustand verschweißt werden.

Die Laminatschichten können beim Verschweißen mittels Vakuum auf der Schweißwalze gehalten werden. Hierzu kann die Schweißwalze Saugöffnungen aufweisen, durch die das Vliesstofflaminat angesaugt wird.

Ausführungsbeispiele der Erfindung werden im Folgenden näher beschrieben. Die schematischen Zeichnungen zeigen in Ausschnitten in
- Fig. 1: einen Querschnitt durch ein erfindungsgemäßes, bahnförmiges Vliesstofflaminat quer zur Bahnlaufrichtung mit unter dem Laminat angeordneter Schweißwalze
- Fig. 2: einen Querschnitt durch das Vliesstofflaminat nach dem Entspannen der elastischen Folie mit schematisch dargestellten Falten der Vliesstoffe
- Fig. 3: eine Draufsicht auf das Vliesstofflaminat nach dem Entspannen
- Fig. 4: eine schematische Darstellung einer Windel
- Fig. 5: eine Draufsicht auf ein aus dem Vliesstofflaminat hergestelltes Windelverschlusselement an einem Windelchassis
- Fig. 6: eine Draufsicht auf die Folienoberfläche im elastischen Bereich im entspannten Zustand nach der Dehnung
- Fig. 7: einen Querschnitt durch die Folie im elastischen Bereich quer zur Längsrichtung im entspannten Zustand nach der Dehnung
- Fig. 8: eine Draufsicht auf die Folienoberfläche im Bereich des Seitenrandes
- Fig. 9: einen Querschnitt durch die Folienoberfläche im Bereich des Seitenrandes quer zur Längsrichtung
- Fig. 10: Ablaufdiagramm für die Herstellung eines Windelverschlusselements

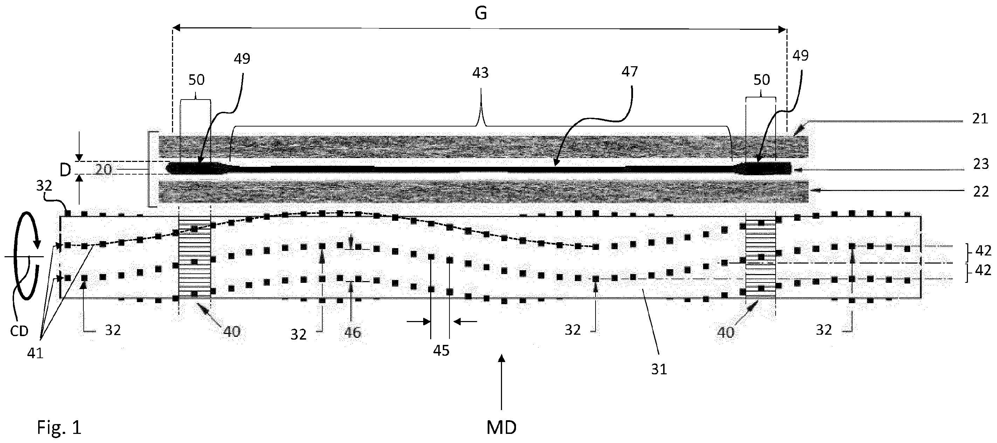

Fig. 1 zeigt ein bahnförmiges Vliesstofflaminat 20, das in dieser Ausführungsvariante zwei Laminatschichten aus Vliesstoff 21, 22 und eine Laminatschicht aus einer elastischen Folie 23 aufweist. Ein alternatives Vliesstofflaminat unterscheidet sich von diesem Vliesstofflaminat 20 dadurch, dass nicht zwei Vliesstoffe sondern nur ein Vliesstoff verwendet wird, so dass nur auf einer Seitenfläche der elastischen Folie 23 ein Vliesstoff 21 anliegt. Die Eigenschaften dieses alternativen Vliesstofflaminats sind jedoch identisch zu dem nachfolgend beschriebenen Vliesstofflaminat 20.

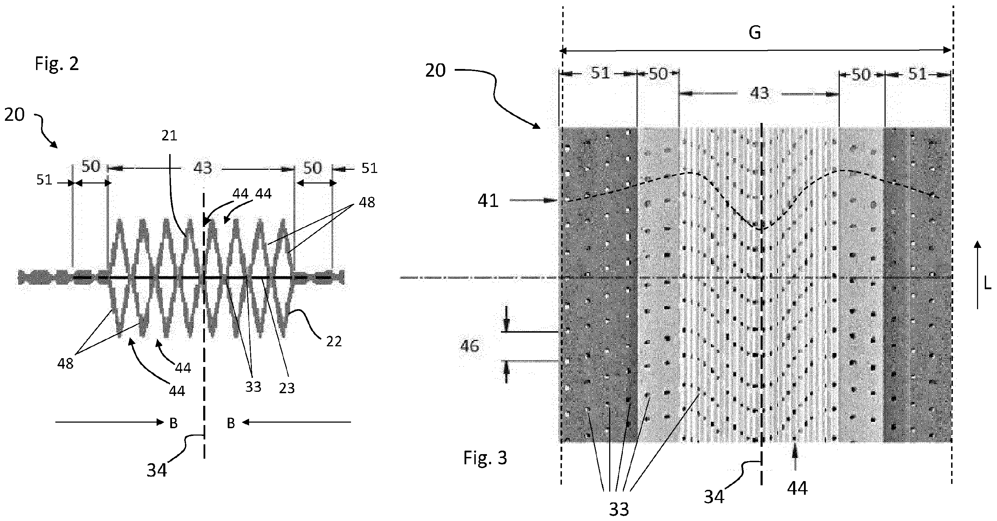

Die Laminatschichten 21, 22, 23 sind in einem gedehnten Zustand der Folie 23 an zahlreichen Schweißpunkten 33 miteinander verschweißt, wobei die Folie 23 beim Verschweißen nur in einem mittleren Bereich 43 quer zur Längsrichtung L des Vliesstofflaminat 20 gedehnt ist und an den beiden seitlichen Längsrändern 50 in einer Breite von jeweils 5 - 25 % der Gesamtbreite G der Folie 23 nicht gedehnt ist. Das Vliesstofflaminat 20 wirft somit im entspannten Zustand der Folie 23, wenn sich diese in Richtung B zur Bahnmitte 34 hin zusammenzieht, im mittleren Bereich 43 Falten 44 im Vliesstoff 21, 22, siehe hierzu Fig. 2. Denn aufgrund der punktuellen Verschweißung bilden sich zwischen zwei benachbarten Schweißpunkten 33 Auftürmungen 48 in den beiden Vliesstoffen 21, 22 und zwischen den Auftürmungen 48 die genannten Falten 44. Die Auftürmungen 48 bilden ein Materialreservoir der Vliesstoffe 21, 22, wodurch das Laminat 20 trotz der nicht elastischen Vliesstoffe im mittleren Bereich 43 insgesamt dehnbar ist. Die Falten 44 erstrecken sich in Längsrichtung L des Vliesstofflaminats 20, wie Figur 3 veranschaulicht.

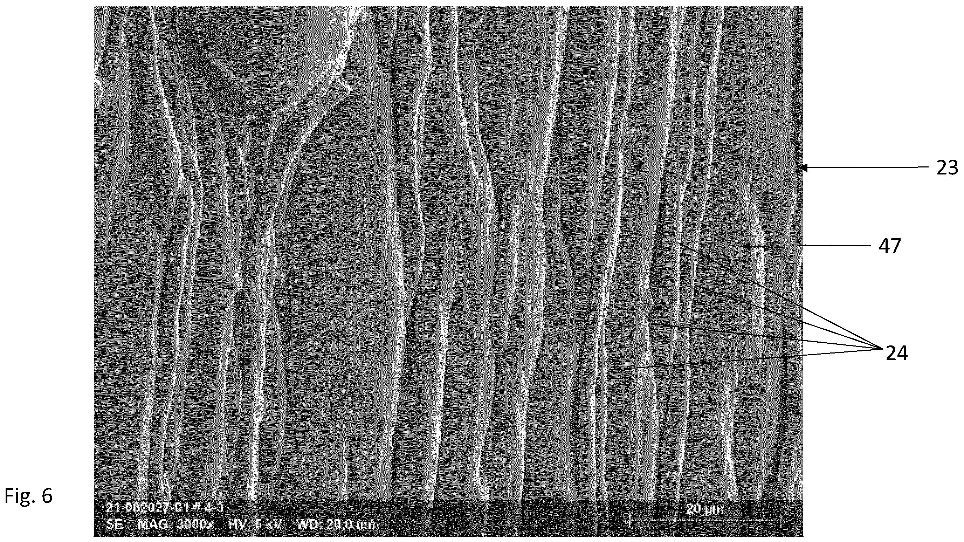

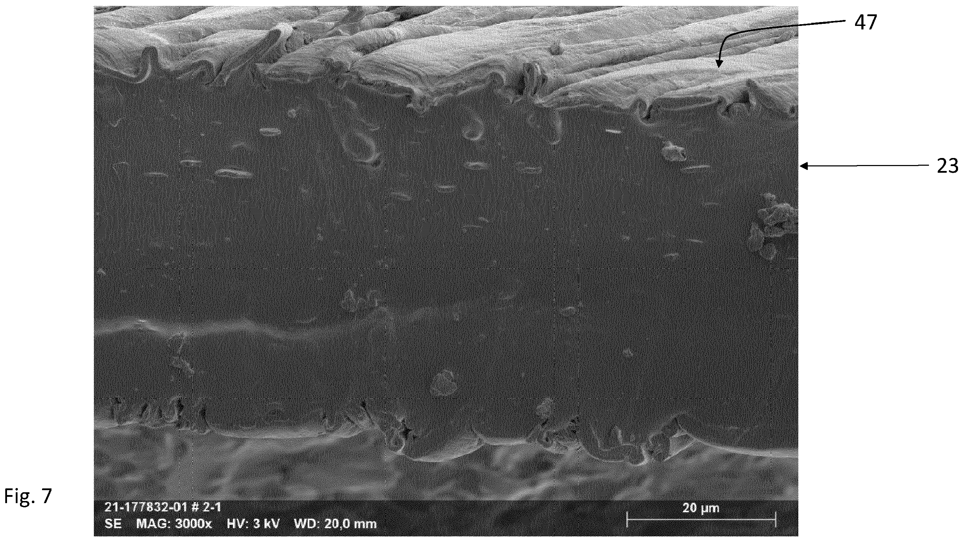

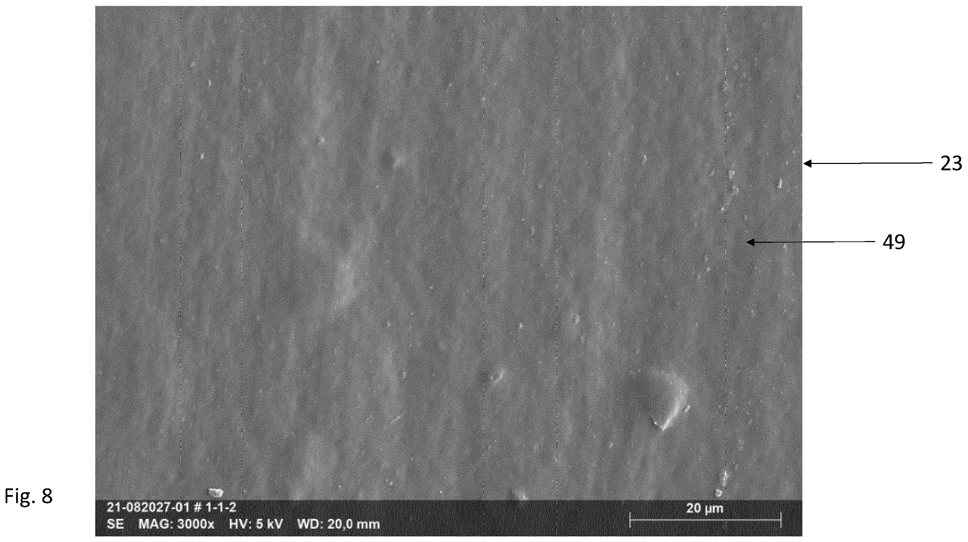

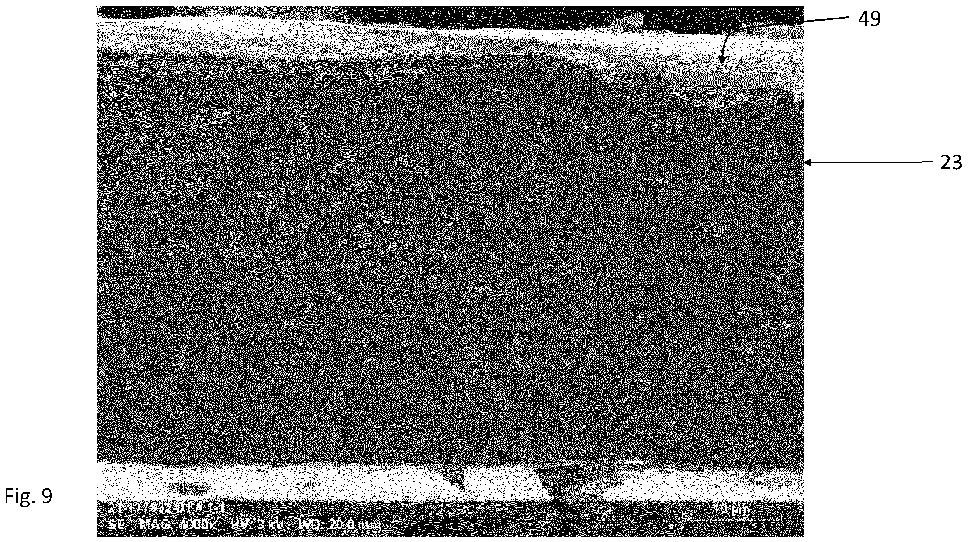

Im Bereich der Längsränder 50, 51 weist das Laminat 20 dagegen keine Falten 44 in den Vliesstoffen 21, 22 auf. Vielmehr bestehen dort parallel zur Bahnmitte 34 verlaufende, nicht elastische Versteifungszonen. Die Folie 23 weist im Bereich der Längsränder 50, d.h. sowohl am rechten als auch am linken Seitenrand 50 eine glatte, geschlossene Oberflächenstruktur 49 auf, die in Figur 8 zu sehen ist, während im mittleren Bereich 43 eine faltige, gefurchte Oberflächenstruktur 47 vorliegt, die in Figur 6 zu sehen ist. Figur 7 zeigt einen Querschnitt durch die Folie 23 im elastischen Bereich 43 quer zur Längsrichtung L. Sie lässt die faltige, gefurchte Oberflächenstruktur 47 ebenfalls erkennen. Entsprechend zeigt Figur 9 einen Querschnitt durch die Folie 23 im Bereich eines Seitenrandes 50 quer zur Längsrichtung L. Sie lässt die glatte, geschlossene Oberflächenstruktur 49 erkennen. Infolge dieser glatten, geschlossenen Oberflächenstruktur 49 und der Tatsache, dass die Längsränder 50 beim Verschweißen nicht gedehnt worden sind, sind die Längsränder 50 als Befestigungszonen besonders geeignet.

In der Ausführungsvariante gemäß Figur 1 erstreckt sich die Folie 23 nur über einen Teil der Gesamtbreite G des Vliesstofflaminats 20, so dass die Laminatschichten aus Vliesstoff 21, 22 die Folie 23 seitlich überragen und in diesem überragenden Bereich einen äußeren Seitenrand 51 bilden, in dem sie direkt aufeinanderliegen. Mit anderen Worten liegt in dem äußeren Seitenrand 51 zwischen den Vliesstoffen 21, 22 keine Folie 23. In Richtung zur Bahnmitte 34 hin liegt zwischen dem mittleren Bereich 43 und den äußeren Seitenrändern 51 jeweils ein innerer Seitenrand 50, in dessen Bereich zwischen den Vliesstoffen 21, 22 der Randbereich der Folie 23 liegt.

Zum Herstellen des bahnförmigen Vliesstofflaminats 20 wird die Folie 23 zwischen den zwei Laminatschichten aus Vliesstoff 23 angeordnet, und in dem mittleren Bereich 43 quer zur Längsrichtung L des Vliesstofflaminats 20 um das 1,5 bis 3-fache (50 bis 200 %), insbesondere um das 2,6 bis 2,8-fache, gedehnt und an den beiden seitlichen Längsrändern 50 in einer Breite von jeweils 5 - 25 % der Gesamtbreite G der Folie 23 nicht gedehnt. Anschließend werden die Laminatschichten 21, 22, 23 bei gedehnter mittiger Folie 23 auf eine Schweißwalze 31 gelegt und in diesem Zustand verschweißt.

Die Schweißwalze 31 ist in Figur 1 unterhalb des Vliesstofflaminats 20 dargestellt. Sie dreht in Bahnlaufrichtung MD. Die Längsrichtung L des Vliesstofflaminats 20 entspricht deren Bahnlaufrichtung MD. Die Achsrichtung der Schweißwalze ist in Figur 1 mit CD gekennzeichnet.

Die Schweißwalze 31 weist Saugöffnungen 40 auf, durch die das Vliesstofflaminat 20 angesaugt, d.h. mittels Vakuum auf der Walze 31 gehalten wird, während das Laminat 20 verschweißt wird. Dies erfolgt mittels Ultraschall.

Die Vliesstoffe 21, 22 bestehen aus Polymerfasern, vorzugsweise aus Polyolefin. Die elastische Folie 23 besteht beispielsweise aus PE (Polyethylen), aus SIS, aus SBS, aus SEBS (Styrol-Ethylen-Butylen-Styrol) oder aus POE (Polyolefin Elastomer).

Die Walze 31 weist erhabene Schweißnoppen 32 auf, die durch eine diesen jenseits des Vliesstofflaminats 20 gegenüberliegende, in Figur 1 nicht dargestellte Sonotrode, die Ultraschallschwingungen ausführt, erhitzt werden, je nachdem, welche Schweißnoppe oder -noppen aufgrund der Drehung der Schweißwalze 31 gegenüberliegen. Aufgrund der Ultraschall-Erwärmung der Schweißnoppen 32 schmilzt die Folie an diesen Stellen, wobei das geschmolzene Material der Folie 23 in die Vliesstoffe 21, 22 eindringt und diese nach der Verfestigung punktuell verklebt, so dass jede Schweißnoppe 32 der Walze 31 einen Schweißpunkt 33 im Vliesstofflaminat erzeugt und die Laminatschichten aneinander geschweißt werden.

Die Schweißnoppen 32 sind in Reihen 41 angeordnet, die quer zur Bahnlaufrichtung MD, mit anderen Worten in Achsrichtung CD der Schweißwalze 31 liegen. Ferner bilden die Schweißnoppen 32 sinusförmige Wellen mit einer Amplitude 42, die das 0,2 bis 0,5-fache des Abstands 46 der Schweißpunktreihen 41 voneinander in Bahnlaufrichtung MD beträgt. Hierbei beträgt der Abstand 46 der Schweißpunktreihen 41 voneinander in Bahnlaufrichtung MD 2mm bis 20 mm vorzugsweise 3mm bis 5 mm. Die Schweißnoppen 32 weisen auf der Schweißwalze 31 innerhalb einer Schweißpunktreihe 41 voneinander einen Abstand 45 von 2mm bis 4 mm, vorzugsweise von 2,2mm bis 2,6 mm auf.

Sobald das Laminat 20 von der Schweißwalze 31 genommen wird, zieht sich die elastische Folie 23 zusammen und erzeugt die Falten 44 in beiden Vliesstoffen 21, 22, d.h. beidseits der Folie 23, wobei die Falten 44 zueinander im Wesentlichen parallel liegen. Aufgrund dieser Falten 44 ist das Laminat dehnfähig, um z. B. in Hygieneprodukten verwendet zu werden, insbesondere als elastischer Windelverschluss 57 in Babywindeln 56.

Im ungedehnten, entspannten Zustand ist die Wellenform aufgrund der geringeren Wellenlänge besonders gut sichtbar, d. h. die wellenförmige Struktur kommt beim Produkt optimal zur Geltung und symbolisiert eine hohe Weichheit und Drapierfähigkeit.

Die Vliesstoffe 21, 22 weisen ein Gewicht von 10 bis 30 g/m² und die elastische Folie 23 ein Gewicht von 30 bis 110 g/m² auf. Die Folie 23 nimmt 25 bis 100 Prozent der gesamten Fläche des Laminats ein.

Die Folie 23 ist eine Coextrusionsfolie mit einer Schichtstruktur der Art A/B/A, wobei die Dicke der Schicht A an den zwei Seitenrändern 50 zwischen 2% - 14 % der Gesamtdicke D der Folie 23 beträgt. Die beiden Seitenränder 50 besitzen jeweils eine Breite von jeweils 5 - 25 % der Gesamtbreite G der Folie 23 und sind nicht gedehnt.

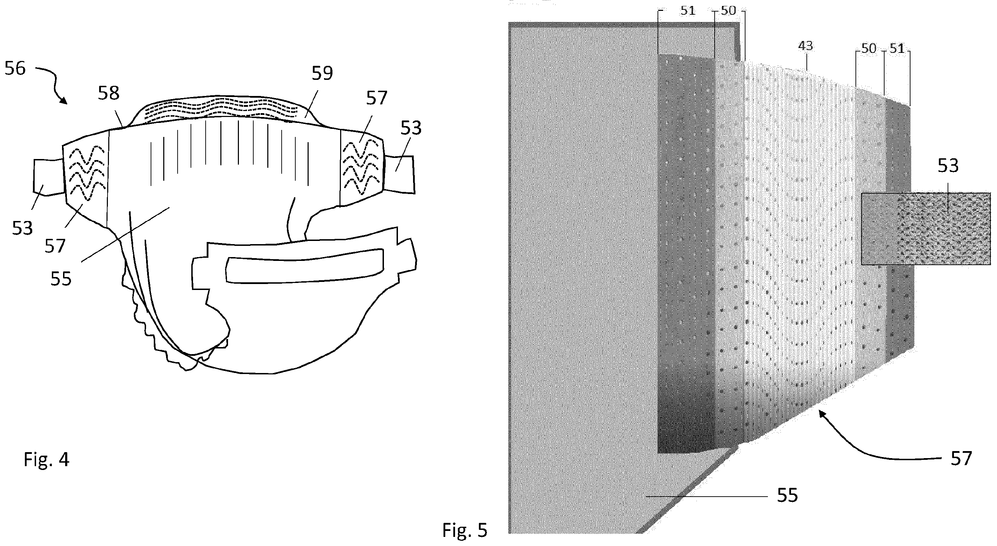

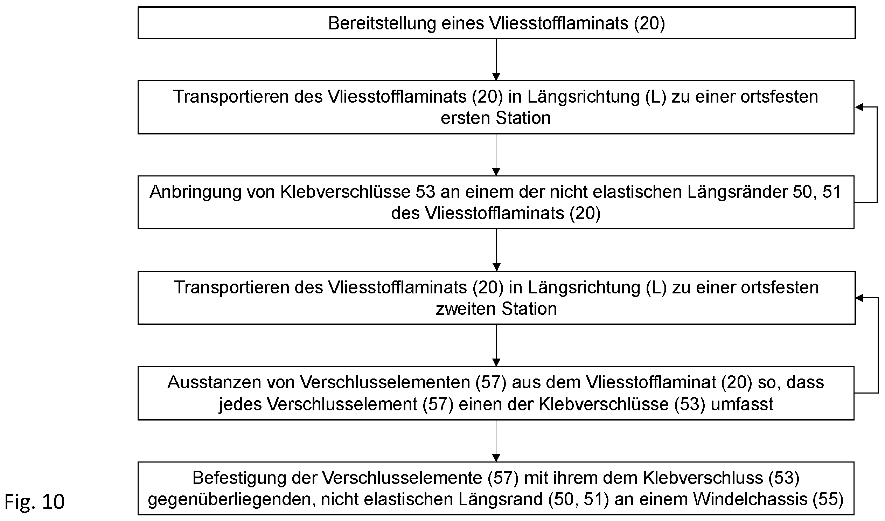

Definition elastisch: Das Laminat 20 wird auf 100 % seiner Länge quer zur Längsrichtung gedehnt und zeigt nach der Dehnung eine elastische Rückstellung von mindestens 80 % und damit eine plastische Verformung von maximal 20 %, genauer im Bereich von 5% - 10%. Eine Messmethode zum Ermitteln der Hysterese ist in der internationalen Anmeldung WO 2018/031841 beschrieben. Wie bereits erläutert, eignet sich das erfindungsgemäße Laminat zur Herstellung eines elastischen Verschlusselements 57 einer Windel 56 oder eines elastisch formgebenden Elements 59 am Rand 58 einer Windel 56, beispielsweise für Babys oder Kleinkinder, wie sie in Figur 4 schematisch dargestellt ist. Figur 5 zeigt den Seitenrand der Windel 56 im Hüftbereich, an dem ein Verschlusselement 57 befestigt ist. Dies erfolgt durch die folgenden Schritte, die auf einer sogenannten Windelmaschine ausgeführt werden können und in Fig. 10 dargestellt sind:
- Bereitstellen eines erfindungsgemäßen, bahnförmigen Vliesstofflaminats 20
- Transportieren des Vliesstofflaminats 20 in Längsrichtung L zu einer ortsfesten ersten Station, an der wiederholt Klebverschlüsse 53 an einem der nicht elastischen Längsränder 50, 51 des Vliesstofflaminats 20 fahnenartig von diesem abstehend angebracht werden, so dass eine Serie von zueinander beabstandeten Klebverschlüssen 53 vorliegt,
- Transportieren des Vliesstofflaminats 20 in Längsrichtung zu einer ortsfesten zweiten Station, an der die gewünschten Verschlusselemente 57 wiederholt aus dem Vliesstofflaminat 20 so ausgestanzt werden, dass jedes Verschlusselement 57 einen der Klebverschlüsse 53 umfasst und
- Befestigung der Verschlusselemente 57 mit ihrem dem Klebverschluss 53 gegenüberliegenden, nicht elastischen Längsrand 50, 51 an einem Windelchassis 55, insbesondere mit Hilfe von Klebstoff und/oder einer Verpressung und/oder Ultraschallschweißen.

Aufgrund der festen, nicht weich elastisch verformbaren Seitenränder 50, 51, die hier die Befestigungszonen bilden, ist die Befestigung der Klebverschlüsse 53 am Vliesstofflaminat 20 einerseits und die Befestigung des aus diesem hergestellten Windelverschlusselements 57 an dem Windelchassis 55 andererseits, besonders einfach herzustellen und prozesssicher.

### Bezugszeichenliste

20 Vliesstofflaminat
21 Erste Laminatschicht aus Vliesstoff
22 Zweite Laminatschicht aus Vliesstoff
23 Elastische Folie
24 Furchen in Folienoberfläche
31 Schweißwalze
32 Schweißnoppen
33 Schweißpunkt
34 Bahnmitte
40 Saugöffnungen
41 Reihe
42 Amplitude der Schweißpunkte
43 Mittlerer Bereich der elastischen Folie
44 Falten im Vliesstoff
45 Noppenabstand entlang einer Reihe
46 Reihenabstand
47 Oberflächenstruktur der Folie innen, faltig, gefurchte
48 Auftürmung
49 Oberflächenstruktur der Folie außen, glatte, geschlossene
50 Innerer Längsrand
51 Äußerer Längsrand
53 Klebverschluss
55 Windelchassis
56 Windel
57 Verschlusselement/ Windelverschluss
58 Rand
59 Elastisch formgebendes Element
MD Bahnlaufrichtung,
CD Achsrichtung der Schweißwalze, senkrecht zu MD
L Längsrichtung
G Gesamtbreite der Folie
D Gesamtdicke der Folie
B Relaxationsrichtung

## Patentansprüche

1. Bahnförmiges Vliesstofflaminat (20) zur Herstellung eines elastischen Verschlusselements (57) einer Windel (56) oder eines elastisch formgebenden Elements (59) am Rand (58) einer Windel (56), mit wenigstens einer Laminatschicht (21, 22) aus Vliesstoff (21, 22) und einer daran anliegenden Laminatschicht (23) aus einer elastischen Folie (23), wobei die Laminatschichten (21, 22, 23) in einem gedehnten Zustand der Folie (23) an zahlreichen Schweißpunkten (33) miteinander verschweißt sind, und die Folie (23) beim Verschweißen in einem mittleren Bereich (43) quer zur Längsrichtung (L) des Vliesstofflaminats (20) gedehnt und an den beiden seitlichen Längsrändern (50) in einer Breite von jeweils 5 - 25 % der Gesamtbreite (G) der Folie (23) nicht gedehnt ist, so dass das Vliesstofflaminat (20) im entspannten Zustand der Folie (23) im mittleren Bereich (43) Falten (44) im Vliesstoff (21, 22) wirft, die sich in Längsrichtung (L) des Vliesstofflaminats (20) erstrecken, und im Bereich der Längsränder (50, 51) keine Falten im Vliesstoff (21, 22) aufweist, sondern dort zwei parallel verlaufende, nicht elastische Versteifungszonen bildet, wobei die Folie (23) im Bereich der Längsränder (50) eine glatte, geschlossene Oberflächenstruktur (49) und im mittleren Bereich (43) eine faltige, gefurchte Oberflächenstruktur (47) aufweist, **dadurch gekennzeichnet, dass** die Folie (23) eine Coextrusionsfolie mit einer Schichtstruktur der Art A/B/A ist, wobei die Dicke der Schicht A zwischen 2% und 14 % der Gesamtdicke (D) der Folie (23) beträgt.

2. Vliesstofflaminat (20) nach Anspruch 1, **dadurch gekennzeichnet, dass** sie zwei Laminatschichten (21, 22) aus Vliesstoff aufweist und die Folie (23) dazwischen liegt.

3. Vliesstofflaminat (20) nach einem der vorherigen Ansprüche, **dadurch gekennzeichnet, dass** die Folie (23) 10% bis 90 % der gesamten Fläche im Vliesstofflaminat (20) einnimmt, bevorzugt 25% bis 50%.

4. Vliesstofflaminat (20) nach einem der vorherigen Ansprüche, **dadurch gekennzeichnet, dass** die Schweißpunkte (33) in sinusförmigen Reihen (41) angeordnet sind, deren Amplituden (42) das 0,2 bis 0,5-fache des Abstands (46) der Reihen (41) voneinander in Längsrichtung (L) des Vliesstofflaminats (20) beträgt.

5. Vliesstofflaminat (20) nach Anspruch 4, **dadurch gekennzeichnet, dass** der Abstand (46) der Schweißpunktreihen (41) zueinander in Längsrichtung (L) des Vliesstofflaminats (20) zwischen 2mm und 20mm vorzugsweise zwischen 3mm bis 5mm beträgt.

6. Vliesstofflaminat (20) nach Anspruch 4 oder 5, **dadurch gekennzeichnet, dass** die Schweißpunkte (33) entlang der Reihen (41) mit einem Abstand (45) zueinander von 2mm bis 4mm, vorzugsweise von 2,2 bis 2,6 mm erzeugt sind.

7. Vliesstofflaminat (20) nach einem der vorherigen Ansprüche, **dadurch gekennzeichnet, dass** die Laminatschicht oder -schichten (21, 22) aus Vliesstoff ein Gewicht von 10g/m² bis 30 g/m² aufweisen.

8. Vliesstofflaminat (20) nach einem der vorherigen Ansprüche, **dadurch gekennzeichnet, dass** die Folie (23) ein Gewicht von 25 g/m² bis 110 g/m² aufweist.

9. Verfahren zum Herstellen des bahnförmigen Vliesstofflaminats (20) nach einem der vorherigen Ansprüche, **dadurch gekennzeichnet, dass** als Folie (23) eine Coextrusionsfolie mit einer Schichtstruktur der Art A/B/A verwendet wird, wobei die Dicke der Schicht A zwischen 2% und 14 % der Gesamtdicke (D) der Folie (23) beträgt und die Folie (23) in einem mittleren Bereich (43) quer zur Längsrichtung (L) des Vliesstofflaminats (20) um das 1,5 bis 3-fache, insbesondere um das 2,6 bis 2,8-fache, gedehnt wird und an den beiden seitlichen Längsrändern (50) in einer Breite von jeweils 5 - 25 % der Gesamtbreite (G) der Folie (23) nicht gedehnt wird, und die Laminatschichten (21, 22, 23) anschließend in diesem Zustand verschweißt werden.

10. Verfahren nach Anspruch 9, **dadurch gekennzeichnet, dass** die Laminatschichten (21, 22, 23) beim Verschweißen mittels Vakuum auf einer Schweißwalze (31) gehalten werden.

11. Verwendung des bahnförmigen Vliesstofflaminats (20) nach einem der Ansprüche 1 bis 8 zur Herstellung von Verschlusselementen (57) für Windeln (56), **gekennzeichnet durch** die Schritte:
- Transportieren des Vliesstofflaminats (20) in Längsrichtung (L) zu einer ortsfesten ersten Station, an der wiederholt Klebverschlüsse (53) an einem der nicht elastischen Längsränder (50, 51) des Vliesstofflaminats (20) fahnenartig von diesem abstehend angebracht werden, so dass eine Serie von zueinander beabstandeten Klebverschlüssen (53) vorliegt,
- Transportieren des Vliesstofflaminats (20) in Längsrichtung zu einer ortsfesten zweiten Station, an der die gewünschten Verschlusselemente (57) wiederholt aus dem Vliesstofflaminat (20) so ausgestanzt werden, dass jedes Verschlusselement (57) einen der Klebverschlüsse (53) umfasst und
- Befestigung der Verschlusselemente (57) mit ihrem dem Klebverschluss (53) gegenüberliegenden, nicht elastischen Längsrand (50, 51) an einem Windelchassis (55), insbesondere mit Hilfe von Klebstoff und/oder einer Verpressung und/oder Ultraschallschweißen.

## Claims

1. A tape-like nonwoven laminate (20) for producing an elastic fastening element (57) of a diaper (56) or an elastic shape-giving element (59) at the edge (58) of a diaper (56), comprising at least one laminate layer (21, 22) of nonwoven fabric (21, 22) and a laminate layer (23) of an elastic film (23) abutting thereto, wherein the laminate layers (21, 22, 23), in a stretched state of the film (23), are welded together at numerous weld points (33), and the film (23) is stretched transverse to the longitudinal direction (L) of the nonwoven laminate (20) in a central region (43) during welding and is not stretched at the two lateral longitudinal edges (50) each over a width of 5-25% of the total width (G) of the film (23), such that the nonwoven laminate (20), when the film (23) is in a relaxed state, forms (43) forms folds (44) in the nonwoven fabric (21, 22) that extend in the longitudinal direction (L) of the nonwoven laminate (20), and in the region of the longitudinal edges (50, 51) does not form any folds in the nonwoven fabric (21, 22), but instead forms two parallel, non-elastic stiffening zones there, wherein the film (23) has a smooth, closed surface structure (49) in the region of the longitudinal edges (50) and a wrinkled, furrowed surface structure (47) in the central region (43), **characterized in that** the film (23) is a coextruded film with an A/B/A layer structure, wherein the thickness of layer A is between 2% and 14% of the total thickness of the film (23).

2. The nonwoven laminate (20) according to claim 1, **characterized in that** it comprises two laminate layers (21, 22) of nonwoven fabric with the film (23) sandwiched between them.

3. The nonwoven laminate (20) according to any of the preceding claims, **characterized in that** the film (23) occupies 10% to 90% of the total area of the nonwoven laminate (20), preferably 25% to 50%.

4. The nonwoven laminate (20) according to any of the preceding claims, **characterized in that** the weld points (33) are arranged in sinusoidal rows (41) whose amplitudes (42) are 0.2 to 0.5 times the distance (46) between the rows (41) in the longitudinal direction (L) of the nonwoven laminate (20).

5. The nonwoven laminate (20) according to claim 4, **characterized in that** the spacing (46) between the rows of weld points (41) in the longitudinal direction (L) of the nonwoven laminate (20) is between 2 mm and 20 mm, preferably between 3 mm and 5 mm.

6. The nonwoven laminate (20) according to claim 4 or 5, **characterized in that** the weld points (33) are formed along the rows (41) at a spacing (45) of 2 mm to 4 mm, preferably 2.2 mm to 2.6 mm.

7. The nonwoven laminate (20) according to any of the preceding claims, **characterized in that** the nonwoven laminate layer or layers (21, 22) have a weight of 10 g/m² to 30 g/m².

8. The nonwoven laminate (20) according to any of the preceding claims, **characterized in that** the film (23) has a weight of 25 g/m² to 110 g/m².

9. A method for manufacturing the web-shaped nonwoven laminate (20) according to any one of the preceding claims, **characterized in that** a coextruded film with an A/B/A layer structure is used as the film (23), wherein the thickness of layer A is between 2% and 14% of the total thickness (D) of the film (23), and the film (23) is stretched in a central region (43) transverse to the longitudinal direction (L) of the nonwoven laminate (20) by a factor of 1.5 to 3, in particular by a factor of 2.6 to 2.8 times, and is not stretched at the two lateral longitudinal edges (50) each over a width of 5-25% of the total width (G) of the film (23), and the laminate layers (21, 22, 23) are subsequently welded together in this state.

10. The method according to claim 9, **characterized in that** the laminate layers (21, 22, 23) are held on a welding roller (31) by vacuum during welding.

11. Use of the tape-like nonwoven laminate (20) according to any one of claims 1 through 8 for the manufacture of fastening elements (57) for diapers (56), **characterized by** the steps of:
- transporting the nonwoven laminate (20) in the longitudinal direction (L) to a stationary first station, at which adhesive fasteners (53) are repeatedly attached in a tab-like manner to one of the non-elastic longitudinal edges (50, 51) of the nonwoven laminate (20) so that a series of spaced-apart adhesive fasteners (53) is formed,
- transporting the nonwoven laminate (20) in the longitudinal direction to a stationary second station, at which the desired fastening elements (57) are repeatedly punched out of the nonwoven laminate (20) such that each fastening element (57) encompasses one of the adhesive fasteners (53), and
- attaching the fastening elements (57) with their non-elastic longitudinal edge (50, 51) opposite the adhesive fastener (53) to a diaper chassis (55), in particular by means of adhesive and/or pressing and/or ultrasonic welding.

## Revendications

1. Un stratifié non tissé (20) en forme de ruban destiné à la fabrication d'un élément de fixation élastique (57) d'une couche-culotte (56) ou d'un élément élastique de mise en forme (59) au niveau du bord (58) d'une couche-culotte (56), comprenant au moins une couche laminée (21, 22) de tissu non tissé (21, 22) et d'une couche stratifiée (23) constituée d'un film élastique (23) venant en butée contre celles-ci, dans lequel les couches stratifiées (21, 22, 23), lorsque le film (23) est étiré, sont soudées entre elles en de nombreux points de soudure (33), et le film (23) est étiré transversalement à la direction longitudinale (L) du stratifié non tissé (20) dans une région centrale (43) pendant le soudage et n'est pas étiré au niveau des deux bords longitudinaux latéraux (50), chacun sur une largeur comprise entre 5 et 25 % de la largeur totale (G) du film (23), de sorte que le stratifié non tissé (20), lorsque le film (23) est à l'état détendu, forme (43) des plis (44) dans le non-tissé (21, 22) qui s'étendent dans la direction longitudinale (L) du stratifié non-tissé (20), et ne forme aucun pli dans le non-tissé (21, 22), mais forme à la place deux zones de renfort parallèles et non élastiques à cet endroit, le film (23) présentant une structure de surface lisse et fermée (49) au niveau des bords longitudinaux (50) et une structure de surface plissée et sillonnée (47) dans la zone centrale (43), **caractérisé en ce que** le film (23) est un film coextrudé présentant une structure en couches A/B/A, l'épaisseur de la couche A étant comprise entre 2 % et 14 % de l'épaisseur totale du film (23).

2. Le stratifié non tissé (20) selon la revendication 1, **caractérisé en ce qu'**il comprend deux couches stratifiées (21, 22) de tissu non tissé entre lesquelles est intercalé le film (23).

3. Le stratifié non tissé (20) selon l'une quelconque des revendications précédentes, **caractérisé en ce que** le film (23) occupe 10 % à 90 % de la surface totale du stratifié non tissé (20), de préférence 25 % à 50 %.

4. Le stratifié non tissé (20) selon l'une quelconque des revendications précédentes, **caractérisé en ce que** les points de soudure (33) sont disposés en rangées sinusoïdales (41) dont les amplitudes (42) sont comprises entre 0,2 et 0,5 fois la distance (46) entre les rangées (41) dans la direction longitudinale (L) du stratifié non tissé (20).

5. Le stratifié non tissé (20) selon la revendication 4, **caractérisé en ce que** l'espacement (46) entre les rangées de points de soudure (41) dans la direction longitudinale (L) du stratifié non tissé (20) est compris entre 2 mm et 20 mm, de préférence entre 3 mm et 5 mm.

6. Le stratifié non tissé (20) selon la revendication 4 ou 5, **caractérisé en ce que** les points de soudure (33) sont formés le long des rangées (41) à un espacement (45) de 2 mm à 4 mm, de préférence de 2,2 mm à 2,6 mm.

7. Le stratifié non tissé (20) selon l'une quelconque des revendications précédentes, **caractérisé en ce que** la ou les couches de stratifié non tissé (21, 22) ont un grammage compris entre 10 g/m² et 30 g/m².

8. Le stratifié non tissé (20) selon l'une quelconque des revendications précédentes, **caractérisé en ce que** le film (23) présente un grammage compris entre 25 g/m² et 110 g/m².

9. Procédé de fabrication du stratifié non tissé en forme de nappe (20) selon l'une quelconque des revendications précédentes, **caractérisé en ce que** l'on utilise comme film (23) un film coextrudé présentant une structure en couches A/B/A, l'épaisseur de la couche A étant comprise entre 2 % et 14 % de l'épaisseur totale (D) du film (23), et le film (23) est étiré dans une zone centrale (43), transversalement à la direction longitudinale (L) du stratifié non tissé (20), d'un facteur compris entre 1,5 et 3, en particulier d'un facteur compris entre 2,6 et 2,8, et n'est pas étiré au niveau des deux bords longitudinaux latéraux (50), chacun sur une largeur comprise entre 5 et 25 % de la largeur totale (G) du film (23), et les couches du stratifié (21, 22, 23) sont ensuite soudées entre elles dans cet état.

10. Procédé selon la revendication 9, **caractérisé en ce que** les couches du stratifié (21, 22, 23) sont maintenues sur un rouleau de soudage (31) par le vide pendant le soudage.

11. Utilisation du stratifié non tissé en forme de bande (20) selon l'une quelconque des revendications 1 à 8 pour la fabrication d'éléments de fixation (57) destinés à des couches-culottes (56), **caractérisée par** les étapes consistant à :
- le transport du stratifié non tissé (20) dans la direction longitudinale (L) vers un premier poste fixe, au niveau duquel des éléments de fixation adhésifs (53) sont fixés de manière répétée, sous forme de languettes, sur l'un des bords longitudinaux non élastiques (50, 51) du stratifié non tissé (20), de sorte qu'une série d'éléments de fixation adhésifs (53) espacés les uns des autres soit formée,
- le transport du stratifié non tissé (20) dans la direction longitudinale vers une deuxième station fixe, au niveau de laquelle les éléments de fixation souhaités (57) sont découpés de manière répétée dans le stratifié non tissé (20) de telle sorte que chaque élément de fixation (57) englobe l'un des éléments de fixation adhésifs (53), et
- fixer les éléments de fixation (57), par leur bord longitudinal non élastique (50, 51) opposé à l'élément de fixation adhésif (53), à une structure de couche (55), notamment au moyen d'un adhésif et/ou par pression et/ou par soudage par ultrasons.
